# EUROPEAN PATENT SPECIFICATION

(11) **EP 0 646 016 B1**
(45) Date of publication and mention of the grant of the patent: **25.08.1999**
(21) Application number: 93916644.3
(22) Date of filing: 18.06.1993
(51) Int. Cl.: A61K 39/00, A61K 38/04, C07K 14/705

(54) **SYNTHETIC PEPTIDES FOR THE TREATMENT OF MYASTHENIA GRAVIS**
SYNTHETISCHE PEPTIDE FÜR DIE BEHANDLUNG VON MYASTHENIA GRAVIS
PEPTIDES SYNTHETIQUES POUR LE TRAITEMENT DE LA MYASTHENIE GRAVE

(30) Priority: 18.06.1992 US 900393
(43) Date of publication of application: 05.04.1995
(73) Proprietor: YEDA RESEARCH AND DEVELOPMENT COMPANY, LTD., Rehovot 76100 (IL)
(72) Inventor: SELA, Michael, 76 100 Rehovot (IL); MOZES, Edna, 76 303 Rehovot (IL)
(74) Representative: VOSSIUS & PARTNER
(86) International application number: US9305906
(87) International publication number: WO9400148

(56) References cited:
- EMBO JOURNAL, vol.8, no.13, 20 December 1989, EYNSHAM, OXFORD GB pages 4049 - 4052 E MOZES ET AL. 'direct binding of a myasthenia gravis related epitope to MHC class II molecules on living murine antigen-presenting cells'
- PROCEEDINGS OF THE NATIONAL ACADEMY OF SCIENCES OF USA., vol.90, no.15, August 1993, WASHINGTON US pages 7000 - 7004 Y KATZ-LEVY ET AL. 'Inhibition of T-cell reactivity to myasthenogenic epitopes of the human acetylcholine receptor by synthetic analogs'
- The Journal of Clinical Investigation, Volume 82, Number 6, issued December 1988, STEFAN BROCKE et al., "In Vitro Proliferative Responses and Antibody Titers Specific to Human Acetylcholine Receptor Synthetic Peptides in Patients with Myasthenia Gravis and Relation to HLA Class II Genes", pages 1894-1900, see especially Table 1.
- The Journal of Immunology, Volume 141, Number 7, issued 01 October 1988, PIETRO PALA et al., "Competition between Unrelated Peptides Recognized by H-2-KD Restricted T Cells", pages 2289-2294, see entire document.
- Immunology, Volume 66, Number 2, issued February 1989, H.C. BODMER et al., "Influenza-Specific Cytotoxic T-Cell Recognition is Inhibited by Peptides Unrelated in Both Sequence and MHC Restriction", pages 163-169, see entire document.
- Proceedings of the National Academy of Science, Volume 86, Number 23, issued December 1989, KOICHIRO SAKAI et al., "Prevention of Experimental Encephalomyelitis with Peptides that Block Interaction of T Cells with Major Histocompatibility Complex Proteins", pages 9470-9474, see entire document.
- Cell, Volume 59, Number 2, issued 20 October 1989, JAMES L. URBAN et al., "Autoimmune T Cells: Immune Recognition of Normal and Variant Peptide Epitopes and Peptide-Based Therapy", pages 257-271, see entire document.
- Cell, Volume 57, issued 02 June 1989, DAVID C. WRAITH et al., "T Cell Recognition as the Target for Immune Intervention in Autoimmune Disease", pages 709-715, see entire document.
- Cell, Volume 59, Number 2, issued 20 October 1989, DAVID C. WRAITH et al., "Antigen Recognition in Autoimmune Encephalomyelitis and the Potential for Peptide-Mediated Immunotherapy", pages 247-255, see entire document.

## Description

The present invention relates to synthetic peptides useful for the treatment of myasthenia gravis (MG) patients, and to pharmaceutical compositions comprising these peptides by themselves, in a polymerized form or attached to a macromolecular carrier.

Autoimmune diseases are characterized by immune responses that are directed against self antigens. These responses are maintained by the persistent activation of self-reactive T lymphocytes. T lymphocytes are specifically activated upon recognition of foreign and/or self antigens as a complex with self Major Histocompatibility Complex (MHC) gene products on the surface of antigen-presenting cells (APC).

Myasthenia gravis (MG) is an autoimmune disorder, the symptoms of which are caused by an antibody-mediated autoimmune attack on the acetylcholine receptor (AChR) of the post-synaptic membrane of the neuromuscular junction. This antibody attack results in loss of acetylcholine receptors and jeopardizes normal neuromuscular transmission, leading to episodic muscle weakness, chiefly in muscles innervated by cranial nerves, and to fatigability.

T lymphocytes are considered to play a central role in the autoreactive process, but the specific immunoregulatory mechanisms by which the T cells exert their regulatory role leading to the induction and various clinical manifestations of MG are poorly understood, and no specific cure is available for the treatment of myasthenic patients. Presently, treatment is with cholinesterase inhibitors, such as pyridostigmine and neostigmine, thymectomy, corticosteroids, and immunosuppressive agents and plasmapheresis.

MG is a well defined autoimmune disorder mediated by antibodies specific to determinants of the AChR. Specific genes of the human MHC, the HLA system, were shown to be significantly associated with the disease. The high frequency of certain histocompatibility antigens (HLA - B8, DR3) in MG patients suggests a defect of immunoregulation that might be expressed on the level of T cells.

In previous studies we found that two peptides representing sequences of the human AChR α-subunit (p195-212 and p257-269) significantly stimulated peripheral blood lymphocytes (PBL) from MG patients in comparison to healthy controls (E. Mozes et al., EMBO J. 8(13):4049-4052; Brocke, S. et al. (1988), J. Clin. Invest. 82:1894-1900). In addition, a correlation was demonstrated between the HLA-DR types of the MG patients and their responses to these peptides. Thus, all patients that expressed HLA-DR3 responded to p257-269 and 83% of patients who expressed HLA-DR5 responded to p195-212.

Extension of this research using inbred mouse strains led to the identification of high, intermediate and low responder strains to the sequences p195-212 and p259-271 of the human AChR α-subunit. Furthermore, lymph node cells, from Torpedo-derived AChR immunized SJL and BALB/c mice, proliferated in response to p195-212 and p259-271, respectively, even better than to the immunizing antigen (Brocke, S. et al. (1990), Immunol. 69:495). These results indicate that peptides p195-212 and p259-271 are immunodominant murine T cell epitopes.

Long-term T cell lines and clones of C3H.SW origin specific to synthetic immunogenic peptides p195-212 and p259-271 were established in our laboratory and described by Brocke, S. et al. (1990a), Internat. Immunol. 2:735-742, herein incorporated by reference. Using these cell lines and clones, it is possible to characterize the T cell recognition process of myasthenic epitopes. Using this method, T cell lines and clones specific to p195-212 were established from lymph node cells of low (C3H.SW) and high (SJL) responder mouse strains, and T cell lines and clones specific to p259-271 were developed from lymph node cells of low (C3H.SW) and high (BALB/c) responder mouse strains.

European patent publication no. 432,691 describes an assay for the measurement of direct binding of a peptide that is a T-cell epitope to gene products of the major histocompatibility complex (MHC), classes I and II, on the surface of intact living antigen-presenting cells (APC). The assay comprises incubating the labelled peptide with the APC and monitoring the extent of binding by the addition of a probe that reacts with the ligand used to label the peptide. The assay is suitable for autoimmune diseases and other immunological disorders. With this assay it was demonstrated that p195-212 binds directly to MHC class II molecules on living APC from several different mouse strains. This observed binding capacity for the peptide was shown to correlate with the proliferative potential of the different mouse strains and was inhibited by the relevant anti-I-A antibodies. In addition, it was shown that APC from MG patients and healthy controls, who responded by proliferation to peptides p195-212 and/or p259-271, also bound the same peptides, labelled with biotin. The ability to screen peptides by their direct binding to MHC products and by their stimulatory capacity to T cells might shed light on the role of MG-related epitopes in the pathogenesis of the disease.

It has been suggested that peptide analogs obtained by amino acid substitutions in a stretch of the sequence of a peptidic antigen relevant to an autoimmune disease might lead to peptides that bind to MHC gene products but that do not stimulate specific helper T cells. Such peptides can be used to inhibit competitively T cell reactivity *in vitro* and *in vivo* and thus for treatment of the corresponding autoimmune disease (Sakai, K. et al. (1989), PNAS 86:9470; Urban, J.L. et al. (1989), Cell 59:257; European patent publication no. 432,691, and PCT publication no. WO 92/04049). However, none of the references provide any information or guidance on the amino acids of the pathogenic peptide that can be substituted and which amino acids can serve as suitable substituents, in order to obtain a peptide analog that will successfully compete with the pathogenic peptide and thus be useful for preventing or treating autoimmune diseases in general, and myasthenia gravis in particular.

It is an object of the present invention to provide synthetic peptides that are analogs of myasthogenic T cell epitopes which represent sequences of the human AChR α-subunit, and are designed for specific treatment of myasthenia gravis. These synthetic peptides bind to MHC Class II gene products but do not activate the T cells involved in MG- related autoimmune responses.

The invention thus relates to synthetic peptides capable of inhibiting the proliferative response of T lymphocytes from myasthenia gravis patients to a myasthogenic peptide corresponding to a sequence of the human acetylcholine receptor α-subunit selected from the peptide p195-212 of the sequence (SEQ ID NO:1): 
195                    200                    205
Asp-Thr-Pro-Tyr-Leu-Asp-Ile-Thr-Tyr-His-Phe-

                210
Val-Met-Gln-Arg-Leu-Pro-Leu
 and the peptide p259-271 of the sequence (SEQ ID NO:2): 
259 260                    265                    270
Val-Ile-Val-Glu-Leu-Ile-Pro-Ser-Thr-Ser-Ser-Ala-Val
 said synthetic peptides having as least nine amino acid residues and differing from the peptides p195-212 and p259-271 by one or more amino acid substitutions, polymers of said peptides and conjugates thereof with a macromolecular carrier.

The response-inhibiting peptides of the invention comprise an amino acid sequence which includes at least amino acid residues 200-208 of SEQ ID NO:1 or amino acid residues 262-266 of SEQ ID NO:2, but differing therefrom by one to three amino acid substitutions, said amino acid substitutions being selected from those which cumulatively do not substantially change the volume, hydrophobic-hydrophilic pattern and charge of the corresponding portion of the unsubstituted myasthogenic peptide wherein 201 Ile, 205 Phe, 206 Val or/and 207 Met of the amino acid residues 200-208 of SEQ ID NO: 1, or 263 Leu, 264 Ile or/and 265 Pro of amino acid residues 262-266 of SEQ ID NO: 2 is/are substituted by a different one of any of the amino acids Met, Gly, Ala, Phe, Val, Leu, Ile, Pro, or Trp;
wherein 202 Thr, 203 Tyr or/and 208 Gln of the amino acid residues 200-208 of SEQ ID No: 1, or 266 Ser of the amino acid residues 262-266 of SEQ ID No: 2 is/are substituted by a different one of any of the amino acids Gln, Ser, Thr, Tyr, Arg, Lys, His, Asn, Asp, or Glu;
wherein 200 Asp or/and 204 His of the amino acid residues 200-208 of SEQ ID NO: 1 or 262 Glu of the amino acid residues 262-266 of SEQ ID No: 2 is/are substituted by a different one of any of the amino acids Ser, Thr, Gln, Tyr, Arg, Lys, His, Asn, Glu or Asp;
wherein a hydrophilic amino acid residue of the amino acid residues 200-208 of SEQ ID NO: 1 or of 262-266 of SEQ ID NO:2 is substituted with a hydrophobic amino acid; or/and
wherein a hydrophobic amino acid residue of the amino acid residues 200-208 of SEQ ID NO: 1 or of 262-266 of SEQ ID NO: 2 is substituted with a hydrophilic amino acid residue.

The invention further relates to pharmaceutical compositions for the treatment of myasthenia gravis by administration of the peptides of the invention by themselves, in a polymerized form or attached to a macromolecular carrier.

### Brief Description of the Drawings

Figure 1 is a graph showing proliferative responses of the T cell line, TCBALB/c259-271, to various doses of the myasthogenic peptide p259-271 and its analogs p305, p306 and p307.
Figure 2 is a graph showing inhibition of the p259-271 specific proliferative response of the T cell line TCBALB/c259-271, with various doses or the inhibitory peptides p305, p306, p307 and p195-212.
Figure 3 is a graph showing specificity or the helper activity of the T cell line, TCBALB/c259-271, with various doses of the peptides (antigen = Ag) p259-271, p305, p306 and p307.
Figure 4 is a graph showing inhibition of the helper activity of the T cell line, TCBALB/c259-271, with various doses of the peptide p259-271 alone or in combination with each of the analogs p305, p306 and p307.
Figure 5 is a graph showing proliferative responses of lymph node cells of BALB/c mice immunized with p259-271, to various doses of p259-271, p305, p306 and p307.
Figure 6 is a graph showing inhibition of the proliferative responses of lymph node cells of BALB/c mice immunized with p259-271, to various doses of p259-271 alone or in combination with p306.
Figure 7 is a graph showing inhibition of the p195-212 specific proliferative response of the T cell line, TCSJL195-212, with two different doses of mixtures of p195-212 with its analog p455.

The present invention relates to analogs or myasthogenic peptides p259-271 (SEQ ID NO:1) and p195-212 (SEQ ID NO:2) which will bind with high affinity to the appropriate MHC Class II molecules but will not lead to further activation of T cells. Examples of such analogs are provided, as is a procedure which may be followed by anyone of ordinary skill in the art in order to identify additional peptides which will also accomplish this function. The invention is based on the design and synthesis of peptides with amino acid substitutions at different positions that are based on parent peptides p195-212 and p259-271. By means of appropriate substitutions, analogs can be identified which are antagonists to the action of the myasthogenic epitopes in the course of myasthenia gravis. An analog which will bind with high affinity to the appropriate MHC Class II molecules but will not lead to further activation of T cells will compete with the myasthogenic peptides which must bind to the same MHC Class II molecules in order to cause activation of the T cells which, in turn, lead to the production of the antibodies which cause myasthenia gravis. By competing with the myasthogenic peptides which cause T cell proliferation, the adverse effects of myasthenia gravis can be ameliorated.

It has been discovered that the portions of the myasthogenic epitopes p259-271 and p195-212 which are most important for their function as a T cell epitope are amino acid residues 200-208 of SEQ ID NO:1 and 262-266 of SEQ ID NO:2. Thus, the changes are preferably made in these core areas. It is expected that the amino acid residues outside of these core areas are not as important to the function of binding to the appropriate MHC Class II molecules and thus it is not expected that changes in these amino acid residues will prevent further activation of the T cells. Accordingly, there is no need to change them, but if substitutions are made in the non-core amino acid residues, they should be selected to fulfill the guidelines discussed herein as to the cumulative effects of the substitutions.

Amino acids may be divided along the lines of volume, hydrophobic-hydrophilic pattern and charge. With respect to volume, those of ordinary skill in the art understand that the amino acids with the largest volume are Trp, Tyr, Phe, Arg, Lys, Ile, Leu, Met and His, while those with the smallest volumes are Gly, Ala, Ser, Asp, Thr and Pro, with others being in between.

With respect to hydrophobic-hydrophilic pattern, it is well known that the amino acids Gly, Ala, Phe, Val, Leu, Ile, Pro, Met and Trp are hydrophobic, whereas all of the remaining amino acids are hydrophilic. Among the hydrophilic amino acids, Ser, Thr, Gln and Tyr have no charge, while Arg, Lys, His and Asn have a positive charge and Asp and Glu have negative charges.

What is important in selecting peptides to be tested for their potential in inhibiting the proliferative response of T lymphocytes from a myasthenia gravis patient to the myasthogenic peptide SEQ ID NO:1 or SEQ ID NO:2 to which it corresponds, is that the substitutions be selected from those which cumulatively do not substantially change the volume, hydrophobic-hydrophilic pattern and charge of the corresponding portion of the unsubstituted myasthogenic peptide. Thus, a hydrophobic residue may be substituted with a hydrophilic residue, or vice-versa, as long as the total effect does not substantially change the volume, hydrophobic-hydrophilic pattern and charge of the corresponding unsubstituted myasthogenic peptide. As indicated above, these substitutions are made in the important epitopic core areas of residues 200-208 of SEQ ID NO:1 and 262-266 of SEQ ID NO:2.

The synthesized analogs of the present invention have a length of at least nine amino acids and preferably from 9-12 amino acids. Each of the analogs preferably has from 1-3 substitutions in the core area.

In a preferred embodiment of the present invention, the substitution or substitutions are selected so as to substitute a hydrophobic amino acid residue in the core area with another hydrophobic amino acid residue selected so as not to cumulatively substantially change the volume of the unsubstituted peptide. For example, in p200-208, the hydrophobic amino acids are 201 Ile, 205 Phe, 206 Val and 207 Met. Each of these may be substituted with any of the other hydrophobic residues in this preferred embodiment. Thus, for example, 207 Met may be substituted with Gly, Ala, Phe, Val, Leu, Ile, Pro or Trp. It has been established that substitution with Ala provides particularly good results. Similarly, in p262-266, 262 Leu, 264 Ile and 265 Pro are all hydrophobic. Thus, from one to three of these can be substituted with another hydrophobic residue so long as the cumulative effect on the volume is not substantial. Thus, for example, the 265 Pro may be substituted by Gly, Ala, Phe, Val, Leu, Ile, Met or Trp. It has been found that substituting the 265 Pro with Phe gives particularly good results.

In a second embodiment, a hydrophilic non-charged residue may be replaced by a charged or non-charged hydrophilic residue. Hydrophilic non-charged residues in the p200-208 peptide include 202 Thr, 203 Tyr and 208 Gln. In the p262-266 peptide, 266 Ser is a non-charged hydrophilic residue. Thus, for example, 208 Gln may be changed to any of Ser, Thr, Tyr, Arg, Lys, His, Asn, Asp or Glu and 266 Ser may be changed to any Thr, Gln, Tyr, Arg, Lys, His, Asn, Asp or Glu. Again, the change must be selected such that the cumulative effect of all changes does not substantially change the volume, hydrophobic-hydrophilic pattern and charge of the corresponding portion of the unsubstituted myasthogenic peptide. Specific examples of changes within this embodiment are the substitution of 208 Gln with Asn or Asp and the substitution of 266 Ser with Lys or Asp.

In another embodiment of the present invention, charged hydrophilic residues are substituted by charged (same or opposite charge) or non-charged hydrophilic residues. In the p200-208 peptide, 200 Asp is a negatively-charged hydrophilic residue and 204 His is a positively-charged hydrophilic residue. In p262-266, 262 Glu is a negatively-charged hydrophilic residue. Thus, for example, 200 Asp may be substituted with Ser, Thr, Gln, Tyr, Arg, Lys, His, Asn or Glu and 262 Glu may be substituted with Ser, Thr, Gln, Tyr, Arg, Lys, His, Asn or Asp. Specific examples may be substituting 200 Asp with Lys (opposite charge) or 262 Glu with Ser (charged to non-charged). Again, whatever substitution is made must be selected so as to not cumulatively substantially change the volume, hydrophobic-hydrophilic pattern and charge of the corresponding portion of the unsubstituted myasthogenic peptide.

Finally, in a further embodiment, it is also possible to replace a hydrophilic residue with a hydrophobic residue or vice-versa, as long as the cumulative effect is within the guidelines. Examples of such substitutions are the substitution of 204 His with Gly or 203 Tyr with Phe or the substitution of 262 Glu with Ala.

Those of ordinary skill in the art of peptide chemistry will readily recognize, by strictly theoretical considerations, what the cumulative effect will be on the charge, hydrophobic-hydrophilic pattern and volume of a given peptide as short as nine amino acids. Thus, it would not take undue experimentation to determine which substitutions should be tried in order to identify analogs in accordance with the present invention which have the function of competing with the native myasthogenic peptides for binding the appropriate MHC Class II molecules but which will not lead to further activation of T cells.

It should be understood that the amino acids which are used for substituting into the native peptide may include modified peptides such as norleucine, hydroxyproline, hydroxylysine, gamma-carboxyglutamic acid, etc.

Some specific substitutions, any 1-3 of which otherwise comply with the guidelines presented herein, may be present in an analog according to the present invention, include the following: 200, Asp-->Lys; 203, Tyr-->Phe;204, His-->Gly; 207, Met-->NLeu; 207, Met-->Ala; 208, Gln-->Asp; 208, Gln-->Asn; 262, Glu-->Asp; 262, Glu-->Lys; 262, Glu-->Ser; 262, Glu--Ala; 265, Pro-->Leu; 265, Pro-->Phe; 266, Ser-->Lys; and 266, Ser-->Asp.

It is expected that the substitution of hydrophilic amino acids, that are mainly involved in T cell receptor interactions, will be effective in blocking T cell activation. Substitutions in the hydrophobic residues may contribute to higher stability of the analog-MHC complexes.

It should be understood that other modifications of the myasthogenic peptides are also contemplated by the present invention. Thus, the peptide of the present invention is intended to include a "chemical derivative" thereof which retains at least a portion of the function of the peptide which permits its utility in preventing or inhibiting T cell proliferative responses and autoimmune disease.

A "chemical derivative" of a peptide of the present invention contains additional chemical moieties not normally a part of the peptide. Covalent modifications of the peptide are included within the scope of this invention. Such modifications may be introduced into the molecule by reacting targeted amino acid residues of the peptide with an organic derivatizing agent that is capable of reacting with selected side chains or terminal residues. Many such chemical derivatives and methods for making them are well known in the art.

Also included in the scope of the invention are salts of the peptides of the invention. As used herein, the term "salts" refers to both salts of carboxyl groups and to acid addition salts of amino groups of the peptide molecule. Salts of a carboxyl group may be formed by means known in the art and include inorganic salts, for example, sodium, calcium, ammonium, ferric or zinc salts, and the like, and salts with organic bases such as those formed for example, with amines, such as triethanolamine, arginine, or lysine, piperidine, procaine, and the like. Acid addition salts include, for example, salts with mineral acids such as, for example, hydrochloric acid or sulfuric acid, and salts with organic acids, such as, for example, acetic acid or oxalic acid. Such chemical derivations would preferably be used to modify the pharmaceutical properties of the peptide insofar as stability, solubility, etc., are concerned.

Once an analog in accordance with the present invention is produced, its ability to inhibit the proliferative response of T lymphocytes to the corresponding myasthogenic peptides may be readily determined by those of ordinary skill in the art without undue experimentation using tests such as those described herein. One test which may be readily conducted is for the ability of substituted peptides to inhibit *in vitro* the proliferative responses of certain T cell lines and clones to the original peptide. The T cell lines and clones are those which have been produced which are specific to the myasthogenic epitopes of SEQ ID NO:1 or SEQ ID NO:2. Another test which can be conducted in order to select analogs having the desired activity is to test for the ability of the substituted peptides to inhibit the ability of the T cell lines and clones to provide help to peptide-specific B cells in the presence of the parent peptide. The substituted peptides may also be tested for their ability to bind directly, following biotinylation, to MHC Class II products on antigen-presenting cells of the relevant strains and to inhibit the binding of the parent myasthogenic epitopes.

Substituted peptides which test positive in one or more of these *in vitro* tests will provide a reasonable expectation of *in vivo* activity. However, *in vivo* tests can also be conducted without undue experimentation. One such *in vivo* animal test would be to immunize naive mice with the myasthogenic peptide ant co-inject the mice with the analogs by various routes and dose schedules. Lymph node cells can then be analyzed for their proliferative potential to the myasthogenic peptides. In addition, titers of anti-AChR antibodies in the sera of these mice can be measured to record the effect of the analogs on the *in vivo* helper cell activity. The advantages of these *in vivo* assays is that, although they are not aimed at assessing the effects of the analogs on disease induction, they allow *in vivo* screening of all analogs in a relatively short time.

As final proof of therapeutic activity, such activity may be directly measured in a murine model *in vivo*. It has previously been shown that some T cell lines and clones specific to the myasthogenic T cell epitopes are capable of inducing MG-related autoimmune manifestations in mice (Kirshner et al. (1994), Cellular Immunology, 157 : 11-28). Therefore, naive mice can be injected with such clones and treated with the selected substitute peptides in order either to prevent or to remit the autoimmune responses. The peptides can be injected into the mice by different routes at different dosages and at different time schedules. In order to determine the pharmacokinetic parameters of the analogs, including volume of distribution, uptake into antigen presenting cells and clearance, one can use biotinylated derivatives of the analogs. The concentration of the soluble fraction of the analogs in the various body fluids can be determined by ELISA, using avidin-coated plates and specific anti-peptide antibodies. Cell bound analogs can be analyzed by FACS, using fluorochrome-conjugated avidin or streptavidin. Furthermore, the treated mice can be tested periodically in order to determine the effect of the peptides on the autoimmune responses and on disease manifestations elicited in the mice by the T cell clones.

It can thus be seen that, besides the preferred embodiments which have been shown to be operable in the examples herein, those of ordinary skill in the art will be able to determine additional analogs which will also be operable following the guidelines presented herein without undue experimentation.

A relatively simple *in vitro* test can also be conducted in order to assay for the expected therapeutic efficacy of any given substituted peptide on any given myasthenia gravis patient. In order to assess the ultimate goal of producing peptides that will bind with high affinity to the appropriate MHC Class II molecules but will not lead to further activation of T cells and will therefore have a therapeutic effect on MG patients, the peptides may be assayed, following biotinylation, for their ability to bind directly to HLA Class II products on antigen-presenting cells in the peripheral blood lymphocytes of the myasthenia gravis patients and to inhibit the binding of the parent myasthogenic epitopes. Healthy control donors and control peptides may be used in such assays to verify their specificity.

A preferred form of the therapeutic agent in accordance with the present invention is the form of a multi-epitope single peptide. Thus, in a preferred embodiment, analogs of each of the two myasthogenic peptides are covalently linked to one another, such as by a short stretch of alanine residues or by a putative site for proteolysis by cathepsin. See, for example, U.S. Patent 5,126,249 and European Patent 495,049 with respect to such sites. This will induce site-specific proteolysis of the preferred form into the two desired analogs. Alternatively, a number of the same or different substituted peptides of the present invention may be formed into a peptide polymer such as, for example, polymerization of the peptides with a suitable polymerization agent, such as 0.1% glutaraldehyde (Audibert et al. (1981), Nature 289:593). The polymer will preferably contain from 5 to 20 peptide residues. Such peptide polymers may also be formed by cross-linking the peptides or attaching multiple peptides to macromolecular carriers. Furthermore, the formulation may simply be a mixture of different peptides in accordance with the present invention.

Suitable macromolecular carriers are, for example, proteins, such as tetanus toxoid, and linear or branched copolymers of amino acids, such as a linear copolymer of L-alanine, L-glutamic acid and L-lysine and a branched copolymer of L-tyrosine, L-glutamic acid, L-alanine and L-lysine (T,G)-A--L, or multichain poly-DL-alanine (M. Sela et al. (1955), J. Am. Chem. Soc. 77:6175). The conjugates with the carriers are obtained, for example, by first coupling the peptide with a water-soluble carbodiimide, such as 1-ethyl-3 (3'-dimethylaminopropyl) carbodiimide hydrochloride, and then performing the conjugation with the macromolecular carrier as described by Muller, G.M. et al. (1982), Proc. Natl. Acad. Sci. USA 79:569. The contents of the coupled peptide in each conjugate are determined by amino acid analysis, in comparison to the composition of the carrier alone.

According to a preferred embodiment of the present invention, one or more active peptides may be attached to a suitable macromolecular carrier or may be polymerized in the presence of glutaraldehyde.

The peptides, polymers thereof or their conjugates with suitable macromolecular carriers, will be given to patients in a form that insures their bioavailability, making them suitable for treatment. If more than one peptide analog is found to have significant inhibitory activity, these analogs will be given to patients in a formulation containing a mixture of the peptides. If an individual patient responds to both pathogenic MG related peptides, namely p195-212 and p259-271, the ultimate treatment will contain the appropriate inhibitory analogs of both peptides in a suitable form.

The invention further includes pharmaceutical compositions comprising at least one synthetic peptide according to the invention, a conjugate thereof with a suitable macromolecular carrier or a polymer thereof optionally with a pharmaceutically acceptable carrier.

The route of administration may include oral, intravenous, subcutaneous, intraarticular, intramuscular, by inhalation, intraperitoneal, intranasal, intrathecal, intradermal, transdermal or other known routes, including the enteral route.

The dose ranges for the administration of the compositions of the present invention are those large enough to produce the desired effect, whereby, for example, an immune response to the myasthogenic peptide, as measured by T cell proliferation *in vitro*, is substantially prevented or inhibited, and further, where the disease is significantly treated. The doses should not be so large as to cause adverse side effects, such as unwanted cross reactions, generalized immunosuppression, anaphylactic reactions and the like.

Effective doses of the peptides of this invention for use in treating an immune-related disease are in the range of about 1 µg to 100 mg/kg body weight. The dosage administered will be dependent upon the age, sex, health, and weight of the recipient, kind of concurrent treatment, if any, frequency of treatment, and the nature of the effect desired.

The synthetic analogs of sequences of the human AChR are aimed at inhibiting or suppressing specific antigen responses of MG patients, without harming other immune responses. This approach is of utmost importance since the currently accepted treatment for MG involves administration of immunosuppressive agents that are both non-specific and have multiple adverse side effects.

The invention will now be illustrated by the following non-limiting examples.

### EXAMPLES

### Example 1. Preparation of the peptides

The synthetic peptides p195-212 and p259-271 and their analogs p455 (207, Met-->Ala); (200, Asp-->Lys); (203, Tyr-->Phe); (204, His-->Gly); (208, Gln-->Asp); p305 (266, Ser--

Asp); p306 (262, Glu-->Lys); p307 (262, Glu-->Asp); (262, Glu-->Ser); and (262, Glu-->Ala) are synthesized by the Merrifield solid phase technique (Merrifield et al. (1963), J. Am. Chem. Soc. 85:2149), with a peptide synthesizer, using commercially available side-chain protected amino acids. Amino acids are added at each step with at least 99% efficiency. The protecting groups are removed and the peptides are cleared from the resin with anhydrous HF.

The peptides are purified by extraction with ethyl acetate or isopropyl acetate and by HPLC. The purity of the peptides is verified by HPLC and by amino acid analysis.

While all of the above peptides have only one substitution each, other peptides having two or three substitutions and retaining the goals of the guidelines presented herein may be synthesized in the same manner.

### Example 2. Inhibition of proliferative responses in vitro of T cell clones to peptide p259-271

T cell lines and clones specific to p259-271 were developed from lymph node cells of high responder BALB/c mice according to the method described by Brocke et al. (1990a), *supra*, and designated TCBALB/c259-271 (Mozes, E. et al. (1991), "Abstracts, 15th International Congress of Biochemistry, Jerusalem, p. 20).

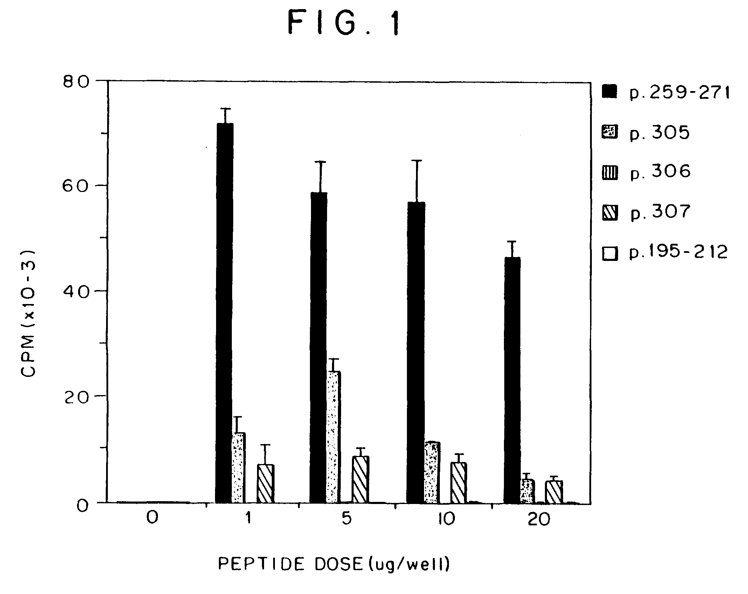

The proliferative response of the T cell clones was assessed by measuring ³H-thymidine incorporation into cells following incubation for the final 16 hours of culture. Cells (10⁴ cells/well) of the T cell line TCBALB/c259-271 were incubated in the presence of irradiated (3000 rad) syngeneic spleen cells from BALB/c mice (0.5 x 10⁶ cells/well) as antigen-presenting cells, in the presence of various concentrations (1, 5, 10, 20 µg/well) of the peptides p195-212, p259-271, p305, p306 and p307. Cultures were set in 0.2 ml RPMI 1640 medium supplemented with 2mM glutamine, 1mM sodium pyruvate, non-essential amino acids, 100 U/ml penicillin, 100 µg/ml streptomycin, 0.25 µg/ml fungizone, 5 x 10⁻⁵ M 2-mercaptoethanol, 10mM HEPES buffer (enriched medium) and 10% fetal calf serum (FCS), for 48 hr, followed by an overnight pulse with ³H-thymidine (0.5 µCi of 5 Ci/mmol). Following incorporation of the isotope, cells were harvested 16 hr later onto a filter paper and radioactivity was determined. The results are shown in Fig. 1 (expressed as mean CPM of triplicates ± SD). Peptides p305 and p307 triggered low proliferative responses of the T cell line at all doses tested (up to 12% and 34.3% respectively, of the response obtained with p259-271), whereas p306 did not stimulate the TCBALB/c259-271 cell line to proliferate. It is not surprising that peptide p195-212 is also shown to competitively inhibit proliferation of T cells specific for peptide p259-271, as p195-212 is directed to the same MHC-determinant as p259-271. Brocke et al. (1990a), *supra*, reports that p195-212 inhibits the proliferative response of the TCSW 259-271 T cell line and p259-271 inhibits the specific proliferative response of the TCSW 195-212 line.

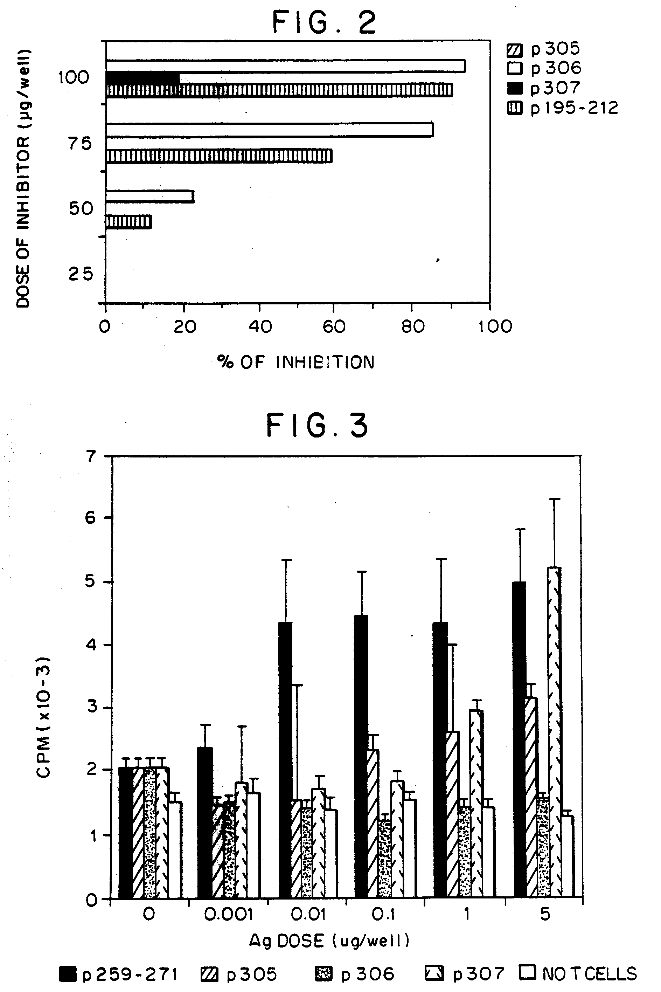

Inhibition of the proliferative responses was performed by addition of increasing doses of the tested substituted peptides (25, 50, 75, 100 µg/well) into the *in vitro* proliferative cultures. Cells (10⁴ cells/well) of the T cell line TCBALB/c259-271, were incubated in the presence of irradiated syngeneic spleen cells (0.5 x 10⁶ cells/well), p259-271 (1 µg/well) and the various doses of the inhibitory peptides p305, p306, p307 and of p195-212, for 48 h. Thereafter, ³H-thymidine (0.5 µCi of 5Ci/mmol) was added, and 16 h later plates were harvested onto a filter paper. Results are expressed as % of inhibition of the p259-271 specific proliferative response. As shown in Figure 2, p306 inhibited up to 93% of the proliferative response of the TCBALB/c259-271 line to p259-271.

### Example 3. In vitro Antibody Production Assay

The synthetic peptides p305, p306 and p307 were examined for their ability to stimulate the TCBALB/c259-271 T cell line to collaborate with peptide-specific B cells in an antibody production assay. B cells, purified from spleens of BALB/c mice previously immunized with 20 µg of p259-271, were cultured (0.5 x 10⁶ cells/well) with normal syngeneic spleen cells (0.5 x 10⁶ cells/well), different doses of p305, p306 and p307 (0.001, 0.01, 0.1, 1 and 5 µg/well) and cells of the TCBALB/c259-271 line (10⁴ cells/well), in 96-well microtiter plates. After 3 days incubation period, medium (enriched RPMI supplemented with 7.5% FCS) was exchanged with fresh medium without antigen, and 4 days later, supernatants were harvested and tested (by solid phase RIA) for the presence of specific anti-p259-271 antibodies. Results are expressed as mean CPM of triplicates ± SD.

As can be seen in Figure 3, low antibody titers were obtained in the presence of p305, whereas the helper activity stimulated by p307 was as efficient as that observed in the presence of the parent peptide. In contrast, no antibody activity could be detected when p306 was added to the culture.

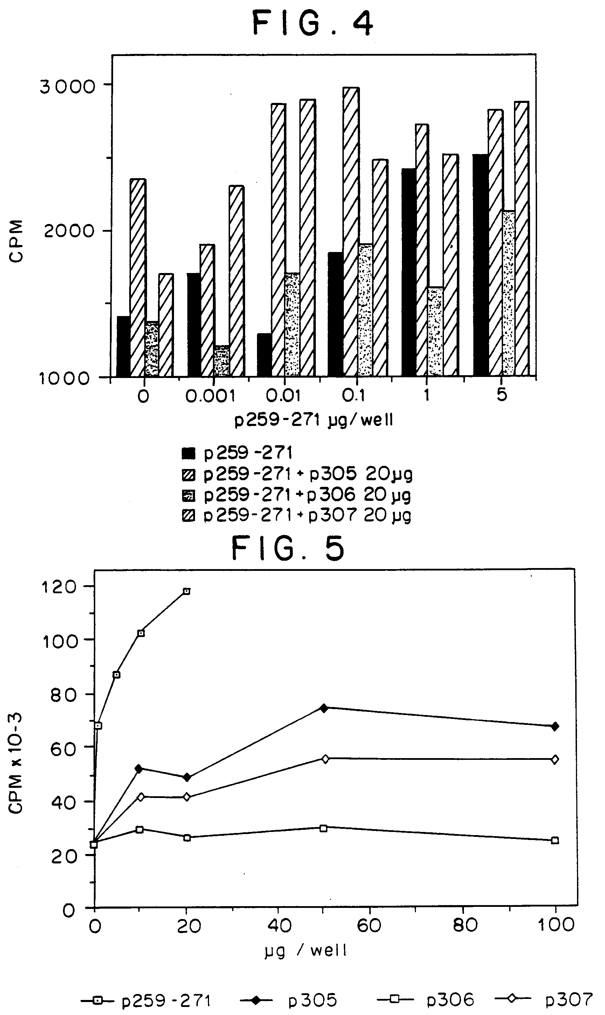

Inhibition of the T cell helper activity was performed by addition of increasing doses of the tested substituted peptides into the *in vitro* culture mixtures. B cells, purified from spleens of BALB/c mice previously immunized with 20 µg of p259-271, were cultured (0.5 x 10⁶ cells/well) with normal syngeneic spleen cells (0.5 x 10⁶ cells/well), different doses of p259-271 alone (0.001, 0.01, 0.1, 1 and 5 µg/well) or together with p305, p306 or p307 (20µg of each) and cells of the TCBALB/c259-271 line (10⁴ cells/well), in 96-well microtiter plates. After 3 days incubation period, medium as above was exchanged with fresh medium, and 4 days later, supernatants were harvested and tested (by solid phase RIA) for the presence of specific anti-p259-271 antibodies. Results are expressed as mean CPM of triplicates ± SD. As shown in Figure 4, p259-271 specific antibody production was inhibited up to 80% in the presence of p306.

### Example 4. Antigen-Specific Proliferative Response of Mouse Lymph Node Cells After Immunization with p259-271

In order to find out whether the peptides p305, p306 and p307 will either stimulate or inhibit the proliferative response of a more heterogeneous T cell population, namely lymph nodes, the following experiments were performed.

The peptides p305, p306 and p307 were examined for their ability to stimulate lymph node cells of p259-271 immunized BALB/c mice to proliferate, in comparison to the parent peptide p259-271. Lymph node cells (0.5 x 10⁶ cells/well) obtained from BALB/c mice immunized with p259-271, were incubated in enriched medium containing 1% normal mouse serum, in the presence of various concentrations of the peptides (10, 20, 50, 100 µg/well) for 96 h. Thereafter, ³H-thymidine (0.5 µCi of 5 Ci/mmol) was added and 16 h later plates were harvested onto a filter paper. Results are expressed as mean CPM of triplicates. As shown in Fig. 5, peptides p305 and p307 triggered low proliferative responses of the cells (up to 53.2% and 32.3% respectively, of that obtained by using p259-271) whereas p306 did not stimulate the lymph node cells to proliferate.

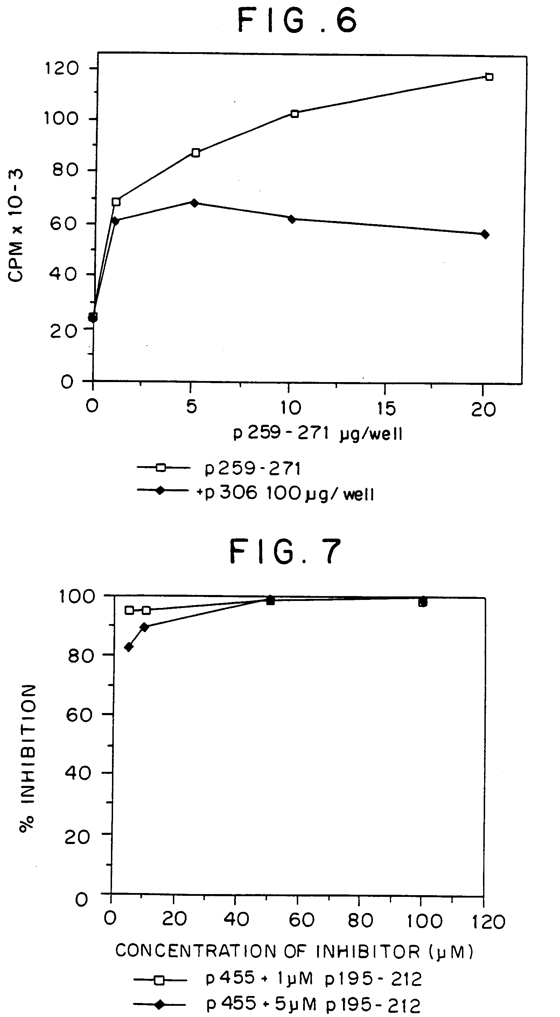

Inhibition of the proliferative responses of lymph node cells of BALB/c mice immunized with p259-271 was done by adding the peptide analog p306 into the incubation mixture at the same time as the pathogenic peptide p259-271. Lymph node cells (0.5 x 10⁶ cells/well) obtained from BALB/c mice immunized with p259-271, were incubated in the presence of various concentrations of p259-271 (1, 5, 10, 20 µg/well) and 100 µg/well of p306 for 96 h. Thereafter, ³H-thymidine (0.5 µCi of 5 Ci/mmol) was added and 16 h later plates were harvested onto a filter paper. Results are expressed as mean CPM of triplicates. As shown in Fig. 6, the peptide p306 inhibited up to 64.7% of the proliferative response of the lymph node cells to p259-271.

### Example 5. Inhibition of Proliferative Responses in vitro of T Cell Clones to Peptide p195-212

T cell lines and clones specific to p195-212 were established from lymph node cells of high (SJL) responder mouse strain by the method described by Brocke et al. (1990a), *supra*, and designated TCSJL195-212 line (Mozes, E. et al. (1991), *supra*).

The proliferative response of the T cell clones was assessed as in the protocol Example 2 which resulted in Fig. 2.

The p195-212 peptide analog, p455, did not stimulate cells of the TCSJL195-212 line to proliferate. Moreover, as shown in Figure 7, p455 inhibited more than 99% of the proliferative response of the TCSJL195-212 line to p195-212. This represents very substantial inhibition.

### Example 6. Inhibition of Proliferation of Human T Cells to the Pathogenic Peptides of Myasthenia Gravis

The action of the pathogenic peptide p259-271 and its analogs p305, p306 and p307, and of p195-212 and its analog p455, on proliferative responses of human T cells was assessed.

Peripheral blood lymphocytes (PBL) of myasthenia gravis patients and of the appropriate control donors (2 x lO⁵ cells/well) were assayed in microtiter plates in 0.2 ml enriched medium containing 10% autologous serum, in the presence of different doses of the peptides p195-212 and the analog p455 for 96h, followed by an overnight pulse with 0.5 µCi of ³H-thymidine. Cells were harvested and radioactivity determined. Inhibition of the proliferative responses was done by adding different doses of p455 into the incubation mixture at the same time as the pathogenic peptide p195-212. Results are reported in Table 1 as (SI) stimulation indices (col. 2 and 3), % inhibition (col. 4).

The PBL of MG patients were tested for their ability to proliferate in the presence of p195-212 and the peptide analog p455. Further, the ability of the analog p455 to inhibit p195-212 specific proliferative responses of the PBL was tested. Table 1 summarizes the responses of three MG patients in these tests. As can be seen in this table, PBL from patients E.K., C.G. and M.R. proliferated in response to p195-212 (SI=6.3, 4.3 and 3.6, respectively), whereas no proliferative responses to the analog were detected at all concentrations (10, 25, 50, 100 µM, corresponding to 20, 50, 100, 200 µg/well) tested. In addition, the analog was able to inhibit the proliferative responses of the PBL from all three patients by 77-100% at an Inhibitor:Stimulator (I:S) ratio of 1:1.

**TABLE 1**

| **Peripheral Blood Lymphocyte Proliferative Responses of Myasthenia Gravis Patients to p195-212 and its Analog p455** | | | |
|---|---|---|---|
| Patient | p195-212 SI* (µg/well)! | Analog SI (µg/well) | % Inhibition (p455:p195-212) |
| E.K. | 6.3 (50) | 1 | 77 (1:1) |
| C.G. | 4.3 (25) | 1 | 100 (1:1) |
| M.R. | 3.6 (25) | 1 | 100 (1:1) |

| | | | |
|---|---|---|---|
| * Stimulation Index = response/background | | | |
| ! Concentration of peptide at SI | | | |

The PBL of MG patients were assayed as above for their ability to proliferate in the presence of different doses of p259-271 and its analogs p305, p306 and p307. In addition, the ability of different doses of the analogs to inhibit p259-271 specific proliferative responses of the PBL was tested. Table 2 summarizes the responses of three MG patients in these tests. As can be seen in this table, PBL from patients I.T., E.K. and C.G. proliferated in response to p259-271 (SI=4.1, 3.7 and 2.7, respectively). Moreover, for each patient, at least one analog was able to inhibit the p259-271 induced proliferative response of the PBL.

**TABLE 2**

| **Peripheral Blood Lymphocyte Response of Myasthenia Gravis Patients to p259-271 and its Analogs** | | | | |
|---|---|---|---|---|
| Patient | p259-271 SI* (µM)! | p305 % Inhibition (I:S)** | p306 % Inhibition (I:S) | p307 % Inhibition (I:S) |
| I.T. | 4.1 (25) | 34.3 (4:1) | 0 | 78 (1:1) |
| E.K. | 3.7 (100) | 82.7 (1:1) | 100 (1:1) | 70 (1:1) |
| C.G. | 2.7 (100) | 85.6 (1:1) | 71.6 (1:1) | 65 (1:1) |

| | | | | |
|---|---|---|---|---|
| * Stimulation Index = response/background | | | | |
| ! Concentration of peptide at SI | | | | |
| ** Inhibitory peptide:Stimulating peptide | | | | |

### Example 7. Induction and Treatment of Experimental MG in Mice

Naive syngeneic mice are inoculated (i.v. in PBS 14 times) with activated peptide specific T cells of the lines TCSJLp195-212 and TCBALB/c259-271 or their derived clones (5-10 x 10⁶ cells). Autoimmune response is determined by serological (e.g., anti murine AChR antibodies), histological and electrophysiological parameters.

An autoimmune response was induced in the inoculated mice as demonstrated by the ability of their sera to stain cells of the C₂ line, a murine muscle cell line that expresses AChR upon differentiation (described in Yaffe, D., et al. (1977), Nature 270 : 725-727; and Inestrosa, N.C., et al. (1983), Exp. Cell. Res. 147 : 393-405). Furthermore, electromyography of the inoculated BALB/c mice revealed a typical myasthenic decrement of the compound muscle action potential (CMAP), that was not observed in the control groups.

In order to prevent or cure the induced experimental MG, peptide analogs are administered a) before inoculation of the peptide specific T cells; b) concomitant with inoculation of the peptide specific T cells; c) after inoculation with the peptide specific T cells but before the appearance of autoimmune responses; d) after the appearance of experimental MG. Reduction in the severity of the autoimmune response indicates suitability of treatment with the peptide.

Reference to known method steps, conventional method steps, known methods or conventional methods is not in any way an admission that any aspect, description or embodiment of the present invention is disclosed, taught or suggested in the relevant art.

The foregoing description of the specific embodiments will so fully reveal the general nature of the invention that others can, by applying knowledge within the skill of the art (including the contents of the references cited herein), readily modify and/or adapt for various applications such specific embodiments, without undue experimentation, without departing from the general concept of the present invention. Therefore, such adaptations and modifications are intended to be within the meaning and range of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance presented herein, in combination with the knowledge of one of ordinary skill in the art.

## Claims

1. A response-inhibiting peptide of at least nine amino acid residues capable of inhibiting the proliferative response of T lymphocytes from a myasthenia gravis patient to a myasthogenic peptide corresponding to a sequence of the human acetylcholine receptor α-subunit selected from the group consisting of peptide p195-212 having the formula of SEQ ID NO:1: 
195 200 205
Asp-Thr-Pro-Tyr-Leu-Asp-Ile-Thr-Tyr-His-Phe-

210
Val-Met-Gln-Arg-Leu-Pro-Leu
 and the peptide p259-271 having the formula of SEQ ID NO:2: 
260                    265                    270
Val-Ile-Val-Glu-Leu-Ile-Pro-Ser-Thr-Ser-Ser-Ala-Val
 said response-inhibiting peptide comprising an amino acid sequence which includes at least amino acid residues 200-208 of SEQ ID NO:1 or amino acid residues 262-266 of SEQ ID NO:2, but differing therefrom by one to three amino acid substitutions, said amino acid substitutions being selected from those which cumulatively do not substantially change the volume, hydrophobic-hydrophilic pattern and charge of the corresponding portion of the unsubstituted myasthogenic peptide wherein 201 Ile, 205 Phe, 206 Val or / and 207 Met of the amino acid residues 200-208 of SEQ ID NO: 1, or 263 Leu, 264 Ile or / and 265 Pro of amino acid residues 262-266 of SEQ ID NO: 2 is / are substituted by a different one of any of the amino acids Met, Gly, Ala, Phe, Val, Leu, Ile, Pro, or Trp;
wherein 202 Thr, 203 Tyr or / and 208 Gln of the amino acid residues 200-208 of SEQ ID No: 1, or 266 Ser of the amino acid residues 262-266 of SEQ ID NO: 2 is/are substituted by a different one of any of the amino acids Gln, Ser, Thr, Tyr, Arg, Lys, His, Asn, Asp, or Glu;
wherein 200 Asp or/and 204 His of the amino acid residues 200-208 of SEQ ID NO: 1 or 262 Glu of the amino acid residues 262-266 of SEQ ID NO: 2 is/are substituted by a different one of any of the amino acids Ser, Thr, Gln, Tyr, Arg, Lys, His, Asn, Glu or Asp;
wherein a hydrophilic amino acid residue of the amino acid residues 200-208 of SEQ ID NO: 1 or of 262-266 of SEQ ID NO:2 is substituted with a hydrophobic amino acid; or/and
wherein a hydrophobic amino acid residue of the amino acid residues 200-208 of SEQ ID NO: 1 or of 262-266 of SEQ ID NO: 2 is substituted with a hydrophilic amino acid residue.

2. A response-inhibiting peptide according to claim 1, conjugated to a macromolecular carrier.

3. A response-inhibiting peptide according to claim 2, wherein the macromolecular carrier is a protein or a copolymer of amino acids.

4. A response-inhibiting peptide according to claim 3, wherein the protein carrier is tetanus toxoid.

5. A response-inhibiting peptide in accordance with any one of claims 1 to 4 having only a single said amino acid substitution.

6. A response-inhibiting peptide in accordance with claim 5, wherein said amino acid substitution is at amino acid 200, 203, 204, 207, 208, 262, 265 or 266.

7. A response-inhibiting peptide in accordance with claim 6, wherein said myasthogenic peptide is peptide p195-212 and said amino acid substitution is at amino acid 200, 203, 204, 207 or 208.

8. A response-inhibiting peptide in accordance with claim 6, wherein said myasthogenic peptide is peptide p259-271 and said amino acid substitution is at amino acid 262 or 266.

9. A response-inhibiting peptide in accordance with any one of claims 1 to 8, wherein said substitutions are selected from the group consisting of:
200, Asp-->Lys; 203, Tyr-->Phe; 204, His-->Gly; 207, Met-->NLeu; 207, Met-->Ala; 208, Gln-->Asp; 208, Gln-->Asn; 262, Glu-->Asp; 262, Glu-->Lys; 262, Glu-->Ser; 262, Glu--Ala; 265, Pro-->Leu; 265, Pro-->Phe; 266, Ser-->Lys; 262, Glu--Ala; 265, Pro-->Leu; 265, Pro-->Phe; 266, Ser-->Lys; and 266, Ser-->Asp.

10. A response-inhibiting peptide in accordance with any of the preceding claims comprising a first amino acid sequence wherein said myasthogenic peptide is p195-212, and a second amino acid sequence wherein said myasthogenic peptide is p259-271, wherein said first and second sequences are linked together.

11. A response-inhibiting peptide in accordance with claim 10, wherein said first and second sequences are linked together by a short stretch of alanine residues.

12. A response-inhibiting peptide in accordance with claim 10, wherein said first and second sequences are linked together by a site for proteolysis by cathepsin.

13. A response-inhibiting peptide in accordance with claim 1, having 9-12 amino acid residues.

14. A peptide polymer capable of inhibiting the proliferative response of T Iymphocytes from a myasthenia gravis patient to a myasthogenic peptide corresponding to a sequence of the human acetylcholine receptor α-subunit selected from the group consisting of peptide p195-212 having the formula of SEQ ID NO:1: 
195 200 205
Asp-Thr-Pro-Tyr-Leu-Asp-Ile-Thr-Tyr-His-Phe-

210
Val-Met-Gln-Arg-Leu-Pro-Leu
 and the peptide p259-271 having the formula of SEQ ID NO:2: 
260 265 270
Val-Ile-Val-Glu-Leu-Ile-Pro-Ser-Thr-Ser-Ser-Ala-Val
 said peptide polymer including an amino acid sequence of at least nine amino acid residues which includes at least amino acid residues 200-208 of SEQ ID NO: 1 or amino acid residues 262-266 of SEQ ID NO: 2, but differing therefrom by one to three amino acid substitutions, said amino acid substitutions being selected from those which cumulatively do not substantially change the volume, hydrophobic-hydrophilic pattern and charge of the corresponding portion of the unsubstituted myasthogenic peptide, said substitutions being as defined in claim 1, said amino acid sequence appearing in said peptide polymer a plurality of times.

15. A peptide polymer in accordance with claim 14, containing from 5 to 20 of said amino acid sequences.

16. A peptide polymer in accordance with claim 14 or 15, wherein said amino acid sequences are linked together by cross-linking.

17. A peptide polymer in accordance with claim 14, containing at least one first amino acid sequence wherein said myasthogenic peptide is p195-212, and at least one second amino acid sequence wherein said myasthogenic peptide is p259-271.

18. A pharmaceutical composition for the treatment of myasthenia gravis comprising an effective amount of a response-inhibiting peptide in accordance with any one of claims 1 to 13 and a pharmaceutically acceptable excipient.

19. A pharmaceutical composition for the treatment of myasthenia gravis comprising an effective amount of a mixture of at least two different peptides in accordance with any one of claims 1 to 13.

20. A pharmaceutical composition for the treatment of myasthenia gravis comprising an effective amount of a peptide polymer in accordance with claim 14 and a pharmaceutically acceptable excipient.

21. A method for selecting response-inhibiting peptides capable of inhibiting the proliferative response of T lymphocytes from a myasthenia gravis patient, comprising:
synthesizing a peptide of a least nine amino acid residues which includes at least amino acid residues 200-208 of SEQ ID NO: 1 or amino acid residues 262-266 of SEQ ID NO: 2, but differing therefrom by one to three amino acid substitutions, said substitutions being selected from those which cumulatively do not substantially change the volume, hydrophobic-hydrophilic pattern and charge of the corresponding portion of the unsubstituted myasthogenic peptide, said substitutions being as defined in claim 1;
testing said peptide for its ability to inhibit the proliferative response of T cells from a myasthenia gravis patient, or a T cell line or clone which is specific to a myasthogenic peptide of SEQ ID NO: 1 or SEQ ID NO: 2 from which said peptide is derived, to the corresponding peptide of SEQ ID NO: 1 or SEQ ID NO: 2 to which the T cells are specific; and
selecting and producing said peptide only if it is capable of inhibiting said proliferative response.

22. A process for the preparation of the peptides of any one of claims 1 to 13 or the peptide polymers of any one of claims 14 to 17 which comprises synthesizing the peptides by solid phase technique using the appropriate side-chain protected amino acids, removing the protecting groups, clearing the peptides from the resin, purifying the peptides and, optionally, polymerizing them.

23. Process for the preparation of the pharmaceutical composition of claims 18 to 20 which comprises combining the peptide(s) of claims 1 to 13 or the peptide polymers of claims 14 to 17 with a pharmaceutically acceptable excipient.

## Patentansprüche

1. Antwort-hemmendes Peptid aus mindestens neun Aminosäureresten, das die proliferative Antwort von T-Lymphocyten eines Patienten mit Myasthenia gravis auf ein myasthogenes Peptid hemmen kann, das einer Sequenz der menschlichen Acetylcholinrezeptor α-Untereinheit entspricht, ausgewählt aus dem Peptid p195-212 mit der Formel der SEQ ID No. 1 
195                    200                    205
Asp-Thr-Pro-Tyr-Leu-Asp-Ile-Thr-Tyr-His-Phe-

                    210
Val-Met-Gln-Arg-Leu-Pro-Leu
 und dem Peptid p259-271 mit der Formel der SEQ ID No. 2, 
260 265 270
Val-Ile-Val-Glu-Leu-Ile-Pro-Ser-Thr-Ser-Ser-Ala-Val
 wobei das Antwort-hemmende Peptid eine Aminosäuresequenz umfaßt, die mindestens die Aminosäurereste 200-208 von SEQ ID No. 1 oder die Aminosäurereste 262-266 von SEQ ID No. 2 einschließt, sich davon aber durch 1 bis 3 Aminosäuresubstitutionen unterscheidet, wobei die Aminosäuresubstitutionen von solchen ausgewählt sind, die insgesamt das Volumen, das hydrophobe-hydrophile Muster und die Ladung des entsprechenden Teils des unsubstituierten myasthogenen Peptids nicht wesentlich verändern, wobei 201 Ile, 205 Phe, 206 Val oder/und 207 Met der Aminosäurereste 200-208 von SEQ ID No. 1, oder 263 Leu, 264 Ile oder/und 265 Pro der Aminosäurereste 262-266 von SEQ ID No. 2 durch eine unterschiedliche Aminosäure von einer der Aminosäuren Met, Gly, Ala, Phe, Val, Leu, Ile, Pro oder Trp substituiert wird/werden;
wobei 202 Thr, 203 Tyr oder/und 208 Gln der Aminosäurereste 200-208 von SEQ ID No. 1 oder 266 Ser der Aminosäurereste 262-266 von SEQ ID No. 2 durch eine unterschiedliche Aminosäure von einer der Aminosäuren Gln, Ser, Thr, Tyr, Arg, Lys, His, Asn, Asp oder Glu substituiert wird/werden;
wobei 200 Asp oder/und 204 His der Aminosäurereste 200-208 von SEQ ID No. 1 oder 262 Glu der Aminosäurereste 262-266 von SEQ ID No. 2 durch eine unterschiedliche Aminosäure von einer der Aminosäuren Ser, Thr, Gln, Tyr, Arg, Lys, His, Asn, Glu oder Asp substituiert wird/werden;
wobei ein hydrophiler Aminosäurerest der Aminosäurereste 200-208 von SEQ ID No. 1 oder von 262-266 von SEQ ID No. 2 durch eine hydrophobe Aminosäure substituiert wird, oder/und
wobei ein hydrophober Aminosäurerest der Aminosäurereste 200-208 von SEQ ID No. 1 oder von 262-266 von SEQ ID No. 2 durch einen hydrophilen Aminosäurerest substituiert wird.

2. Antwort-hemmendes Peptid nach Anspruch 1 in Konjugation mit einem makromolekularen Träger.

3. Antwort-hemmendes Peptid nach Anspruch 2, wobei der makromolekulare Träger ein Protein oder ein Copolymer aus Aminosäuren ist.

4. Antwort-hemmendes Peptid nach Anspruch 3, wobei der Proteinträger ein Tetanustoxoid ist.

5. Antwort-hemmendes Peptid nach einem der Ansprüche 1 bis 4, das nur eine einzige Aminosäuresubstitution aufweist.

6. Antwort-hemmendes Peptid nach Anspruch 5, wobei die Aminosäuresubstitution an der Aminosäure 200, 203, 204, 207, 208, 262, 265 oder 266 ist.

7. Antwort-hemmendes Peptid nach Anspruch 6, wobei das myasthogene Peptid das Peptid p195-212 und die Aminosäuresubstitution an der Aminosäure 200, 203, 204, 207 oder 208 ist.

8. Antwort-hemmendes Peptid nach Anspruch 6, wobei das myasthogene Peptid das Peptid p259-271 und die Aminosäuresubstitution an der Aminosäure 262 oder 266 ist.

9. Antwort-hemmendes Peptid nach einem der Ansprüche 1 bis 8, wobei die Substitutionen ausgewählt sind aus:
200, Asp-->Lys; 203, Tyr-->Phe; 204, His-->Gly; 207, Met-->NLeu; 207, Met-->Ala; 208, Gln-->Asp; 208, Gln-->Asn; 262, Glu-->Asp; 262, Glu-->Lys; 262, Glu-->Ser; 262, Glu-->Ala; 265, Pro-->Leu; 265, Pro-->Phe; 266, Ser-->Lys; und 266, Ser-->Asp.

10. Antwort-hemmendes Peptid nach einem der vorhergehenden Ansprüche, das eine erste Aminosäuresequenz, in der das myasthogene Peptid p195-212 ist, und eine zweite Aminosäuresequenz umfaßt, in der das myasthogene Peptid p259-271 ist, wobei die erste und zweite Sequenz miteinander verbunden sind.

11. Antwort-hemmendes Peptid nach Anspruch 10, wobei die erste und zweite Sequenz durch einen kurzen Abschnitt aus Alaninresten miteinander verbunden sind.

12. Antwort-hemmendes Peptid nach Anspruch 10, wobei die erste und zweite Sequenz durch eine Stelle für die Proteolyse durch Cathepsin miteinander verbunden sind.

13. Antwort-hemmendes Peptid nach Anspruch 1, das 9 bis 12 Aminosäurereste aufweist.

14. Peptidpolymer, das die proliferative Antwort von T-Lymphocyten eines Patienten mit Myasthenia gravis auf ein myasthogenes Peptid hemmen kann, das einer Sequenz der menschlichen Acetylcholinrezeptor α-Untereinheit entspricht, ausgewählt aus dem Peptid p195-212 mit der Formel der SEQ ID No. 1 
195                    200                    205
Asp-Thr-Pro-Tyr-Leu-Asp-Ile-Thr-Tyr-His-Phe-

                    210
Val-Met-Gln-Arg-Leu-Pro-Leu
 und dem Peptid p259-271 mit der Formel der SEQ ID No. 2, 
260                    265                    270
Val-Ile-Val-Glu-Leu-Ile-Pro-Ser-Thr-Ser-Ser-Ala-Val
 wobei das Peptidpolymer eine Aminosäuresequenz aus mindestens neun Aminosäureresten umfaßt, die mindestens die Aminosäurereste 200-208 von SEQ ID No. 1 oder die Aminosäurereste 262-266 von SEQ ID No. 2 einschließt, sich davon aber durch ein bis drei Aminosäuresubstitutionen unterscheidet, wobei die Aminosäuresubstitutionen aus solchen ausgewählt sind, die insgesamt das Volumen, das hydrophobe-hydrophile Muster und die Ladung des entsprechenden Teils des unsubstituierten myasthogenen Peptids nicht wesentlich verändern und wie in Anspruch 1 definiert sind, und wobei die Aminosäuresequenz in dem Peptidpolymer mehrere Male erscheint.

15. Peptidpolymer nach Anspruch 14, das 5 bis 20 Aminosäuresequenzen enthält.

16. Peptidpolymer nach Anspruch 14 oder 15, wobei die Aminosäuresequenzen durch Vernetzung miteinander verbunden sind.

17. Peptidpolymer nach Anspruch 14, das mindestens eine erste Aminosäuresequenz, in der das myasthogene Peptid p195-212 ist, und mindestens eine zweite Aminosäuresequenz enthält, in der das myasthogene Peptid p259-271 ist.

18. Arzneimittel zur Behandlung von Myasthenia gravis, das eine wirksame Menge eines Antwort-hemmenden Peptids nach einem der Ansprüche 1 bis 13 und einen pharmazeutisch verträglichen Exzipienten umfaßt.

19. Arzneimittel zur Behandlung von Myasthenia gravis, das eine wirksame Menge eines Gemisches von mindestens zwei unterschiedlichen Peptiden nach einem der Ansprüche 1 bis 13 umfaßt.

20. Arzneimittel zur Behandlung von Myasthenia gravis, das eine wirksame Menge eines Peptidpolymers nach Anspruch 14 und einen pharmazeutisch verträglichen Exzipienten umfaßt.

21. Verfahren zum Auswählen von Antwort-hemmenden Peptiden, die die proliferative Antwort von T-Lymphocyten eines Patienten mit Myasthenia gravis hemmen können, umfassend:
Synthese eines Peptids aus mindestens neun Aminosäureresten, das mindestens die Aminosäurereste 200-208 von SEQ ID No. 1 oder die Aminosäurereste 262-266 von SEQ ID No. 2 einschließt, sich davon aber durch ein bis drei Aminosäuresubstitutionen unterscheidet, wobei die Aminosäuresubstitutionen aus solchen ausgewählt sind, die insgesamt das Volumen, das hydrophobe-hydrophile Muster und die Ladung des entsprechenden Teils des unsubstituierten myasthogenen Peptids nicht wesentlich verändern und wie in Anspruch 1 definiert sind;
Testen das Peptids auf seine Fähigkeit zur Hemmung der proliferativen Antwort von T-Zellen eines Patienten mit Myasthenia gravis oder einer T-Zellinie oder eines T-Zellclons, die oder der für das myasthogene Peptid SEQ ID No. 1 oder SEQ ID No. 2 spezifisch ist, von dem das Peptid stammt, auf das entsprechende Peptid SEQ ID No. 1 oder SEQ ID No. 2, für das die T-Zellen spezifisch sind; und
Auswählen und Herstellen des Peptids nur dann, wenn es zur Hemmung der proliferativen Antwort fähig ist.

22. Verfahren zur Herstellung von Peptiden nach einem der Ansprüche 1 bis 13 oder von Peptidpolymeren nach einem der Ansprüche 14 bis 17, umfassend die Synthese der Peptide mittels Festphasentechnik unter Verwendung von geeigneten, an den Seitenketten geschützten Aminosäuren, die Entfernung der Schutzgruppen, das Ablösen der Peptide von dem Harz, die Reinigung der Peptide und gegebenenfalls ihre Polymerisation.

23. Verfahren zur Herstellung des Arzneimittels nach einem der Ansprüche 18 bis 20, umfassend die Kombination des Peptids oder der Peptide nach einem der Ansprüche 1 bis 13 oder der Peptidpolymere nach einem der Ansprüche 14 bis 17 mit einem pharmazeutisch verträglichen Excipienten.

## Revendications

1. Peptide inhibiteur de réponse d'au moins neuf restes aminoacides capables d'inhiber la réponse proliférative des lymphocytes T d'un patient atteint de myasthénie grave speudo-paralytique ("myasthenia gravis") à un peptide myasthogène correspondant à une séquence de la sous-unité α du récepteur humain de l'acétylcholine et choisi parmi l'ensemble constitué par le peptide p195-212 répondant à la formule de SEQ ID No. 1 : 
195                 200                 205
Asp-Thr-Pro-Tyr-Leu-Asp-Ile-Thr-Tyr-His-Phe-

                210
Val-Met-Gln-Arg-Leu-Pro-Leu
 et le peptide p259-271 répondant à la formule de SEQ ID No. 2 : 
260                 265                 270
Val-Ile-Val-Glu-Leu-Ile-Pro-Ser-Thr-Ser-Ser-Ala-Val
 ledit peptide inhibiteur de réponse comprenant une séquence aminoacide qui comprend au moins les restes aminoacides 200-208 de SEQ ID No. 1 ou les restes aminoacides 262-266 de SEQ ID No. 2, mais en différant par un à trois remplacements d'aminoacide, lesdits remplacements d'aminoacide étant choisis parmi ceux qui cumulativement ne modifient essentiellement pas le volume, le motif hydrophobe-hydrophile et la charge de la portion correspondante du peptide myasthogène non-modifié où 201 Ile, 205 Phe, 206 Val et/ou 207 Met des restes aminoacides 200-208 de SEQ ID No. 1, ou 263 Leu, 264 Ile et/ou 265 Pro des restes aminoacides 262-266 de SEQ ID No. 2 est/sont remplacé(s) par un aminoacide différent parmi l'un quelconque des aminoacides Met, Gly, Ala, Phe, Val, Leu, Ile, Pro, ou Trp ;
où 202 Thr, 203 Tyr et/ou 208 Gln des restes aminoacides 200-208 de SEQ ID No. 1, ou 266 Ser des restes aminoacides 262-266 de SEQ ID No. 2 est/sont remplacé(s) par un aminoacide différent parmi l'un quelconque des aminoacides Gln, Ser, Thr, Tyr, Arg, Lys, His, Asn, Asp, ou Glu ;
où 200 Asp et/ou 204 His des restes aminoacides 200-208 de SEQ ID No. 1, ou 262 Glu des restes aminoacides 262-266 de SEQ ID No. 2 est/sont remplacé(s) par un aminoacide différent parmi l'un quelconque des aminoacides Ser, Thr, Gln, Tyr, Arg, Lys, His, Asn, Glu ou Asp;
où un reste aminoacide hydrophile des restes aminoacides 200-208 de SEQ ID No. 1, ou 262-266 de SEQ ID No. 2 est remplacé par un reste aminoacide hydrophobe ; et/ou
où un reste aminoacide hydrophobe des restes aminoacides 200-208 de SEQ ID No. 1, ou 262-266 de SEQ ID No. 2 est remplacé par un reste aminoacide hydrophile.

2. Peptide inhibiteur de réponse suivant la revendication 1, conjugué à un véhicule macromoléculaire.

3. Peptide inhibiteur de réponse suivant la revendication 2, dans lequel le véhicule macromoléculaire est une protéine ou un copolymère d'aminoacides.

4. Peptide inhibiteur de réponse suivant la revendication 3, dans lequel le véhicule protéinique est un toxoïde tétanique.

5. Peptide inhibiteur de réponse suivant l'une quelconque des revendications 1 à 4, ayant un seul desdits remplacements d'aminoacides.

6. Peptide inhibiteur de réponse suivant la revendication 5, dans lequel ledit remplacement aminoacide intervient en position 200, 203, 204, 207, 208, 262, 265 ou 266.

7. Peptide inhibiteur de réponse suivant la revendication 6, dans lequel ledit peptide myasthogène est le peptide p195-212 et ledit remplacement aminoacide intervient en position 200, 203, 204, 207 ou 208.

8. Peptide inhibiteur de réponse suivant la revendication 6, dans lequel ledit peptide myasthogène est le peptide p259-271 et ledit remplacement aminoacide intervient en position 262 ou 266.

9. Peptide inhibiteur de réponse suivant l'une quelconque des revendications 1 à 8, dans lequel lesdits remplacements sont choisis parmi l'ensemble constitué par :
200, Asp → Lys ; 203, Tyr → Phe ; 204, His → Gly ; 207, Met → Nleu ; 207, Met → Ala ; 208, Gln → Asp ; 208, Gln → Asn ; 262, Glu → Asp ; 262, Glu → Lys ; 262, Glu → Ser ; 262, Glu → Ala ; 265, Pro → Leu ; 265, Pro → Phe ; 266, Ser → Lys ; et 266, Ser → Asp.

10. Peptide inhibiteur de réponse suivant l'une quelconque des revendications précédentes comprenant une première séquence d'aminoacides où ledit peptide myasthogène est p195-212, et une seconde séquence d'aminoacides où ledit peptide myasthogène est p259-271, et dans lequel lesdites première et seconde séquences sont liées ensemble.

11. Peptide inhibiteur de réponse suivant la revendication 10, dans lequel lesdites première et seconde séquences sont liées ensemble par un petit allongement de restes alanine.

12. Peptide inhibiteur de réponse suivant la revendication 10, dans lequel lesdites première et seconde séquences sont liées ensemble par un site de protéolyse par la cathepsine.

13. Peptide inhibiteur de réponse suivant la revendication 1, ayant 9 à 12 restes aminoacides.

14. Polymère peptidique capable d'inhiber la réponse proliférative des lymphocytes T d'un patient atteint de myasthénie grave speudo-paralytique ("myasthenia gravis") à un peptide myasthogène correspondant à une séquence de la sous-unité α du récepteur humain de l'acétylcholine et choisi parmi l'ensemble constitué par le peptide p195-212 répondant à la formule de SEQ ID No. 1 : 
195                     200                     205
Asp-Thr-Pro-Tyr-Leu-Asp-Ile-Thr-Tyr-His-Phe-

                210
Val-Met-Gln-Arg-Leu-Pro-Leu
 et le peptide p259-271 répondant à la formule de SEQ ID No. 2 : 
260 265 270
Val-Ile-Val-Glu-Leu-Ile-Pro-Ser-Thr-Ser-Ser-Ala-Val
 ledit polymère peptidique comprenant une séquence d'au moins 9 résidus aminoacides qui comprend au moins les restes aminoacides 200-208 de SEQ ID No. 1 ou les restes aminoacides 262-266 de SEQ ID No. 2, mais en différant par un à trois remplacements d'aminoacide, lesdits remplacements d'aminoacide étant choisis parmi ceux qui cumulativement ne modifient essentiellement pas le volume, le motif hydrophobe-hydrophile et la charge de la portion correspondante du peptide myasthogène non-modifié, lesdits remplacements étant tels que définis dans la revendication 1, ladite séquence d'aminoacides apparaissant plusieurs fois dans ledit peptide polymère.

15. Polymère peptidique suivant la revendication 14, contenant de 5 à 20 séquences aminoacides.

16. Polymère peptidique suivant la revendication 14 ou 15, dans lequel lesdites séquences aminoacides sont liées ensemble par réticulation.

17. Polymère peptidique suivant la revendication 14, contenant au moins une première séquence aminoacide où le peptide myasthogène est p195-212, et au moins une seconde séquence aminoacide où le peptide myasthogène est p259-271.

18. Composition pharmaceutique pour le traitement de la myasthénie grave speudo-paralytique ("myasthenia gravis") comprenant une quantité efficace d'un peptide inhibiteur de réponse selon l'une quelconque des revendications 1 à 13 et un excipient pharmaceutiquement acceptable.

19. Composition pharmaceutique pour le traitement de la myasthénie grave speudo-paralytique comprenant une quantité efficace d'un mélange d'au moins deux peptides différents suivant l'une quelconque des revendications 1 à 13.

20. Composition pharmaceutique pour le traitement de la myasthénie grave speudo-paralytique comprenant une quantité efficace d'un polymère peptidique selon la revendication 14 et un excipient pharmaceutiquement acceptable.

21. Procédé pour sélectionner des peptides inhibiteurs de réponse capables d'inhiber la réponse proliférative des lymphocytes T d'un patient atteint de myasthénie grave speudo-paralytique, ledit procédé comprenant :
la synthèse d'un peptide ayant au moins neuf restes aminoacides et qui comprend au moins des restes aminoacides 200-208 de SEQ ID No. 1 ou des restes aminoacides 262-266 de SEQ ID No. 2, mais en différant par un ou trois remplacements d'aminoacide, lesdits remplacements d'aminoacide étant choisis parmi ceux qui cumulativement ne modifient essentiellement pas le volume, le motif hydrophobe-hydrophile et la charge de la portion correspondante du peptide myasthogène non-modifié, lesdits remplacements étant tels que définis dans la revendication 1 ;
l'essai dudit peptide pour son aptitude à inhiber la réponse proliférative de cellules T d'un patient atteint de myasthénie grave speudoparalytique, d'une lignée de cellules T ou d'un clone qui est spécifique du peptide myasthogène selon SEQ ID No. 1 ou SEQ ID No. 2, d'où dérive ledit peptide, à un peptide correspondant de SEQ ID No. 1 ou de SEQ ID No. 2 pour lequel lesdites cellules T sont spécifiques; et,
la sélection et la production dudit peptide si et seulement si il est capable d'inhiber ladite réponse proliférative.

22. Procédé pour la préparation des peptides selon l'une quelconque des revendications 1 à 13 ou des polymères peptidiques selon l'une quelconque des revendications 14 à 17, ledit procédé comprenant la synthèse des peptides selon la technique dite en phase solide en utilisant les aminoacides appropriés à chaîne latérale protégée, l'élimination des groupes protecteurs, la séparation des peptides d'une résine, la purification des peptides et, le cas échéant, leur polymérisation.

23. Procédé pour la préparation de la composition pharmaceutique selon les revendications 18 à 20, ledit procédé comprenant la combinaison du/des peptide(s) des revendications 1 à 13 ou des polymères peptidiques des revendications 14 à 17 avec un excipient pharmaceutiquement acceptable.


SEQUENCE LISTING

(1) GENERAL INFORMATION:

(i) APPLICANT: SELA, MICHAEL
MOZES, EDNA

(ii) TITLE OF INVENTION: SYNTHETIC PEPTIDES FOR THE
TREATMENT OF MYASTHENIA GRAVIS

(iii) NUMBER OF SEQUENCES: 2

(iv) CORRESPONDENCE ADDRESS:
(A) ADDRESSEE: Browdy & Neimark
(B) STREET: 419 Seventh Street, N.W., Suite 300
(C) CITY: Washington
(D) STATE: D.C.
(E) COUNTRY: USA
(F) ZIP: 20004

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER: US 07/900,393
(B) FILING DATE: 18-JUN-1992
(C) CLASSIFICATION:

(viii) ATTORNEY/AGENT INFORMATION:
(A) NAME: Browdy, Roger L.
(B) REGISTRATION NUMBER: 25,618
(C) REFERENCE/DOCKET NUMBER: SELA=3A

(ix) TELECOMMUNICATION INFORMATION:
(A) TELEPHONE: (202)628-5197
(B) TELEFAX: (202)737-3528
(C) TELEX: 248633

(2) INFORMATION FOR SEQ ID NO:1:

(i) SEQUENCE CHARACTERISTICS:
(A) LENGTH: 18 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:1:

Asp Thr Pro Tyr Leu Asp Ile Thr Tyr His Phe Val Met
1               5                   10

Gln Arg Leu Pro Leu
    15

(2) INFORMATION FOR SEQ ID NO:2:

(i) SEQUENCE CHARACTERISTICS:
(A) LENGTH: 13 amino acids
(B) TYPE: amino acid
(C) STRANDEDNESS: single
(D) TOPOLOGY: linear

(ii) MOL[?][?]ULE TYPE: peptide

(xi) SEQUENCE DESCRIPTION: SEQ ID NO:2:

Val Ile Val Glu Leu Ile Pro Ser Thr Ser Ser Ala Val
1               5                   10